# EUROPEAN PATENT SPECIFICATION

(11) **EP 3 102 288 B1**
(45) Date of publication and mention of the grant of the patent: **16.05.2018**
(21) Application number: 15701737.7
(22) Date of filing: 22.01.2015
(51) Int. Cl.: A61N 5/06

(54) **CAMERA-BASED MONITORING OF VITAL SIGNS OF DURING PHOTOTHERAPY TREATMENT**
KAMERABASIERTE ÜBERWACHUNG VON LEBENSZEICHEN IN DER PHOTOTHERAPIEBEHANDLUNG
SURVEILLANCE DE SIGNES VITAUX SUR LA BASE DE CAMÉRAS PENDANT LE TRAITEMENT PAR PHOTOTHÉRAPIE

(30) Priority: 05.02.2014 EP 14153928
(43) Date of publication of application: 14.12.2016
(73) Proprietor: Koninklijke Philips N.V., 5656 AE Eindhoven (NL)
(72) Inventor: KIRENKO, Ihor Olehovych, 5656 AE Eindhoven (NL)
(74) Representative: Ledeboer, Johannes Albertus
(86) International application number: PCT/EP2015/051178
(87) International publication number: WO 2015/117828

(56) References cited:
- WO-A1-2009/073396
- US-A1- 2008 269 849
- US-A1- 2012 150 264
- WIM VERKRUYSSE ET AL: "Remote plethysmographic imaging using ambient light", OPTICS EXPRESS, vol. 16, no. 26, 22 December 2008 (2008-12-22), page 21434, XP055065281, ISSN: 1094-4087, DOI: 10.1364/OE.16.021434

## Description

### FIELD OF THE INVENTION

The present invention relates to a device and a method for remotely obtaining vital sign information of a living being under phototherapy treatment. The present invention further relates to a phototherapy apparatus for treating a living being with light.

### BACKGROUND OF THE INVENTION

Jaundice refers to the yellow appearance of the skin that occurs with the deposition of bilirubin in the dermal and subcutaneous tissue. Normally in the body, bilirubin is processed through the liver, where it is conjugated to glucuronic acid. This conjugated form of bilirubin is then excreted into the bile and removed from the body via the gut. When this excretion process is low following birth, does not work efficiently, or is overwhelmed by the amount of endogenously produced bilirubin, the amount of bilirubin in the body increases, resulting in hyperbilirubinemia and jaundice. Jaundice occurs in as many as 60% of all normal newborns within the first week of life. Effective treatments to decrease bilirubin levels in infants with severe jaundice include phototherapy. Thereby, phototherapy refers to the use of visible light for the treatment of hyperbilirubinemia in the newborn. This relatively common therapy lowers the serum bilirubin level by transforming bilirubin into watersoluble isomers that can be eliminated without conjugation in the liver. The dose of phototherapy largely determines how quickly it works; the dose, in turn, is determined by the wavelength of the light, the intensity of the light (irradiance), the distance between the light and the infant, and the body surface area exposed to the light.

Other application areas of phototherapy (sometimes also referred to as light therapy) include treatment of skin disorders (e.g. psoriasis), sleep disorders and some psychiatric disorders. Commercially available phototherapy systems include those that deliver light via fluorescent bulbs, halogen quartz lamps, light-emitting diodes, and fiberoptic mattresses.

The monitoring of the respiratory rate and other vital signs (sometimes also called biometrical signals) such as the heart rate and the blood oxygen saturation (SpO2) is an important issue in neonatal care (Juliann M. Di Fiore, "Neonatal cardiorespiratory monitoring techniques", Seminars in Neonatology (2004) 9, 195-203.). An approach to unobtrusive vital sign monitoring includes using a video camera and extracting vital sign information from recorded images. One technique in this field that has been demonstrated and found relevant for patient monitoring is remote photoplethysmography (RPPG). RPPG technology is, for instance, described in Wim Verkruysse, Lars O. Svaasand, and J. Stuart Nelson, "Remote plethysmographic imaging using ambient light", Optics Express, Vol. 16, No. 26, December 2008. It is based on the principle that temporal variations in blood volume in the skin lead to variations in light absorptions by the skin. Such variations can be registered by a video camera that takes images of a skin area, e.g. the face, and by calculating a pixel average signal over a selected region (e.g. a part of the cheek of a patient). By looking at periodic variations of this average signal, the heart beat rate and respiratory rate (the respiratory rate may also be referred to as breathing rate or as respiration rate) can be extracted. By evaluating reflections of light of different wavelengths it is also possible to extract the blood oxygen saturation. Meanwhile, there are a number of publications and patent applications that describe details of devices and methods for obtaining vital signs of a patient by use of RPPG.

Another approach for respiratory rate monitoring based on recorded images includes the evaluation of observed periodic movements of patterns or irregularly illuminated parts of the chest of a human being that allow deriving movements of the chest. This is usually referred to as motion analysis.

In WO 2009/073396 A1, an apparatus and method for providing patient access in a warming therapy device (e.g., incubator, warmer, etc.) is described. In one exemplary embodiment, the apparatus includes at least one phototherapy light device disposed so as to provide phototherapy to an infant patient disposed within the warming therapy device, and at least one camera disposed to permit viewing of the infant patient during the phototherapy treatment. In another exemplary embodiment, the apparatus also includes at least one examination light in addition to the phototherapy light device, for assisting with examinations of the infant patient.

In US 2012/0150264 A1 a skin radiation apparatus and method are presented. The apparatus comprises: a photon radiation unit for generating a line-shaped radiation pattern extending in a first direction, the photon radiation unit comprising a photon radiation source, a movement facility for moving the line shaped radiation pattern in a second direction transverse to the first direction, a detection unit for detecting a skin condition profile, a control unit for controlling an power density distribution for the line-shaped radiation pattern dependent on the skin condition profile detected by the detection unit.

In US 2008/0269849 A1 an apparatus for delivering phototherapy includes at least one substrate, at least one emitter mounted on the substrate, and which emits at least two peak wavelengths of light, and an electronic circuit that controls emitter timing. The apparatus is configured as a dressing. A corresponding method includes delivering a first pulse of light to the target tissue from the emitter with a peak wavelength of light, and delivering at least a second pulse of light having a peak wavelength of light that is different from the peak wavelength of the first pulse of light, and the steps define a method of delivering a series of pulse sets of light, and the first and second pulses of light define a pulse set of light.

Although regular video data have been shown to yield adequate vital signs in many cases, the image acquisition for challenging cases, like strong motion, low light levels, non-white illumination, needs further improvement. In camera-based approaches, the extracted vital sign signal is usually very small and hidden in much larger variations due to ambient illumination changes and motion. Moreover, some RPPG techniques rely on availability of illumination in the range of wavelengths, which correspond to Blue, Red, Green (for RGB-based PPG) and Red, InfraRed (for R-IR PPG) color, with more or less equal distribution of an intensity across the spectrum of the light. This makes it difficult to apply RPPG in phototherapy applications where abnormal light conditions are present.

### SUMMARY OF THE INVENTION

It is an object of the present invention to provide a device and a computer program for remotely obtaining vital sign information of a living being under phototherapy treatment. It is further an object of the present invention to provide a phototherapy apparatus.

In a first aspect of the present invention a device for remotely obtaining vital sign information of a living being under phototherapy treatment is presented. The device comprises:
a detection unit for detecting light of at least one wavelength interval reflected from at least a region of interest of the living being and for generating an input signal from the detected light;
a processing unit for processing the input signal and deriving vital sign information of said living being from said input signal by use of remote photoplethysmography and/or motion analysis; and
an illumination unit interface for communicating with an illumination unit for providing the phototherapy treatment to the living being; and
a control unit for controlling at least one of the detection unit and the illumination unit based on the configuration of the detection unit and based on the configuration of the illumination unit.

In another aspect of the present invention, there are provided a computer program which comprises program code means for causing a computer to perform the steps of a method for remotely obtaining vital sign information of a living being under phototherapy treatment as outlined below when said computer program is carried out on a computer, as well as a non-transitory computer-readable recording medium that stores therein a computer program product, which, when executed by a processor, causes said method to be performed.

The method comprises the steps of:
detecting with a detection unit light of at least one wavelength interval reflected from at least a region of interest of the living being and generating an input signal from the detected light;
processing the input signal and deriving vital sign information of said living being from said input signal by use of remote photoplethysmography and/or motion analysis;
communicating via an illumination unit interface with an illumination unit (12) for providing the phototherapy treatment to the living being (14); and
controlling at least one of the detection unit and an illumination unit for providing the phototherapy treatment to the living being based on the configuration of the detection unit and based on the configuration of the illumination unit.

In yet another aspect, a phototherapy apparatus for treating a living being with light is presented. This apparatus comprises:
an illumination unit for providing phototherapy treatment to a living being;
a device for remotely obtaining vital sign information of the living being as described above; and
a user interface for outputting the obtained vital sign information.

Preferred embodiments of the invention are defined in the dependent claims. It shall be understood that the claimed method and apparatus have similar and/or identical preferred embodiments as the claimed device and as defined in the dependent claims.

If a living being (baby, adult or animal) is under phototherapy treatment, it is often difficult or impossible to apply camera based vital sign monitoring techniques because the treatment light (therapeutic light) influences the detected light. Herein, the portion of a phototherapy apparatus actually generating the treatment light, i.e. delivering the treatment to the living being, is referred to as illumination unit. Considering the various application areas of phototherapy, this illumination unit can generally correspond to any light emitting device such as a LED, an array of LEDs of the same or different colors, one or more filament bulbs, one or more neon lamps, a laser, a fluorescent lamp or combinations thereof. This illumination unit illuminates the living being or at least a part of the living being and provides the phototherapy.

The present invention allows remote vital sign monitoring while the living being to be monitored is currently under phototherapy treatment.

The detection unit as used in a device according to the present invention may correspond to a photodetector such as a CMOS, CCD etc. The detection unit is used for detecting light (i.e. receiving light) that is reflected from a region of interest of the living being, i.e. capture images of said region of interest. Usually a series of images is captured over time, i.e. the detection unit operates a certain frame sampling time (sometimes also referred to as frame rate). The region of interest may correspond to a small area on the forehead of a person or also to a larger area on the chest of a person. The light is detected and an input signal is generated that can then be used for extracting vital sign information. The input signal may particularly be extracted from a sequence of captured images of the region of interest. More precisely, the input signal may correspond to the light intensity values detected for one pixel or from averaging a plurality of pixels.

The actual vital sign extraction based on this input signal is performed in a processing unit. This processing unit implements RPPG techniques and/or motion analysis techniques to derive vital sign information. RPPG particularly refers to extracting the heart rate, the respiratory rate and/or the blood oxygen saturation from variations in the reflected light that are indicative of volumetric changes of the blood or a blood vessel of the living being. Motion analysis particularly refers to extracting the respiratory rate from movements of the living that can be deduced from evaluating the reflected light, i.e. the obtained images of the living being. For instance, a distinct line in an image of a region of interest of a living being including the belly or the chest will be bent (i.e. subject to deformation) when the chest or belly is moved due to breathing of the living being.

Both the detection unit and the illumination unit are subject to control, wherein control can simply refer to powering the units on or off or also to more complex control operations such as the adjustment of switching frequencies, frame sampling times, intensity, orientation etc. The control unit considers the configuration of the detection unit as well as the configuration of the illumination unit and determines based thereupon optimal control parameters in order to improve the extraction of vital sign information. On the one hand it is possible to adapt the settings of the detection unit (i.e. to control the detection unit) to the configuration of the illumination unit on the other it is also possible to adapt the settings of the illumination unit (i.e. to control the illumination unit) to the configuration of the detection unit. The control is usually based on the configuration of both the detection unit and the illumination unit. Therein, the configuration of the detection unit may refer to adjustable parameters such as the frame sampling time at which the light is detected, the resolution, the orientation, the adjustment or alignment, the focusing, etc., but also to invariable properties such as the physical resolution, the model number or the spectral sensitivity of an image sensor, the angle of view etc. The configuration of the illumination unit may refer to adjustable parameters such as the intensity, the orientation, the illumination frequency etc., as well as to invariable properties such as the type of light source, the geometry, etc.

An element of the present invention is to control at least one of the detection unit and the illumination unit based on the configuration of the detection unit and the illumination unit. By means of applying a control that considers the configuration of the two, the negative impact of the strong light of a phototherapy unit on RPPG measurements can be mitigated. Furthermore, the robustness of the measurements can even be increased by exploiting advantages of a dedicated illumination (i.e. by controlling the illumination unit in order to deliver light that is suitable for the extraction of vital sign information, e.g. by means of RPPG an/or motion analysis). Controlling herein particularly refers to adapting the settings of the units. In contrast to previous remote vital sign monitoring approaches the present invention can therefore deliver an improved quality of vital sign information of a living being under phototherapy treatment.

According to an embodiment, the device as described above comprises an illumination unit interface for communicating with the illumination unit (uni- or bidirectional communication), i.e. for obtaining information on the configuration of the illumination unit and/or for controlling the illumination unit. This interface allows a comfortable and immediate control of the illumination unit and makes it possible that the configuration can be accessed comfortably and changes of the configuration can directly be observed.

In a preferred embodiment the illumination unit includes an array of LEDs and the control unit is configured to control the array of LEDs to produce a light structure on the region of interest of the living being. Such an array of LEDs offers a plurality of control options. Illuminating a subset of the LEDs can allow projecting a light pattern, i.e. a light structure (sometimes also referred to as structured light) that exhibits one or more light-dark transitions, on the region of interest of the living being. This allows the detection unit to capture high contrast images of the region of interest. Such high contrast images can be advantageous for extracting vital sign information, in particular for extracting the respiratory rate based on analyzing an observed motion or movement in the image. Also, a specifically selected light pattern may allow improving the quality (i.e. the signal-to-noise ratio) of the input signal forming the basis for the vital sign information.

Further preferably, the processing unit is configured to determine changes of the light structure on the region of interest of the living being based on the detected light; and derive the respiratory rate of the living being based on evaluating the determined changes of the light structure. A high contrast image, in particular an image exhibiting at least one light-dark transition, i.e. a distinct line, can be used as a basis for a geometrical approach to determine the respiratory rate of a living being. If, e.g., a region of interest on the chest of a living being is observed, it may become possible to observe changes or movements, in particular periodic changes or movements, of said distinct line via image processing techniques and deduce the respiratory rate of the living being therefrom.

Further, according to another embodiment, the illumination unit includes an array of LEDs and the control unit is configured to control the array of LEDs to produce a recurrent first light structure alternating with a recurrent second light structure on the region of interest of the living being, said alternating light structures in particular alternating at an alternation frequency >50 Hz. One disadvantage of illuminating the region of interest with a light structure is that only a part of the living being is illuminated. Thus, albeit it is possible to extract the respiratory rate as described above, a lower dose of phototherapy treatment is provided. Also, providing a constant light structure may have the effect that only some skin areas are treated. This could result in a less efficient or even ineffective treatment. This disadvantage can be overcome by producing alternating light structures. If, e.g., two alternating (periodic) light structures are produced, the second light structure can illuminate those parts of the region of interest of the living being (and of the remaining surface of the living being) that are not illuminated by the first light structure. Thereby, it is also possible to produce more than two alternating light structures. By producing light structures that alternate at a frequency > 50 Hz, a human eye will not observe the fluctuating light and the living being receiving the treatment will not be disturbed. It is also possible that a time period of no illumination is inserted between the alternating light structures. A further advantage thereof is that the robustness or the quality of the extracted respiratory rate can be increased. Usually the frame sampling time of the detection unit will be adapted to the frequency of the alternating light structures.

In another embodiment, the control unit is further configured to control the detection unit to operate at a frame sampling time corresponding to the alternation frequency of the alternating light structures. If the detection unit is configured to detect the light, i.e. to capture an image, every time another light structure is produced, alternating light structures may allow increasing the sample rate for the input signal. Thereby a more robust input signal may be obtained and the vital sign information may be extracted at a higher accuracy. An image is captured of every light structure (i.e. every time the structure is alternated) on the region of interest. As described above, the respiratory rate can be deduced by evaluating the movements of the light structure from one frame to another.

According to a preferred embodiment, the illumination unit includes an array of LEDs and the control unit is configured to control the array of LEDs to periodically illuminate the region of interest of the living being with at least a subset of the LEDs in said array of LEDs. This array of LEDs may correspond to an array of high energy, blue light LEDs as usually used for providing phototherapy treatment to newborns suffering from hyperbilirubinemia. Also, it may be possible that the array of LEDs includes LEDs of one or more other colors in addition to the high energy LEDs. Advantageously, it then becomes possible to alternatingly illuminate the living being with phototherapy light and other light. Then, the detection unit can be controlled to detect reflected light at times, when no high energy light is present.

In another embodiment, the control unit is configured to control the detection unit to operate at a frame sampling time being adapted to a flashing frequency of the illumination unit. The general concept of aligning the frame sampling time of the detection unit, i.e. the camera sampling, with a flashing frequency of the illumination unit, i.e. an illumination frequency at which the illumination unit provides the phototherapy light, can basically be used in combination with any type of illumination unit. Thereby, it is possible to avoid that negative effects of the phototherapy light on the light that is captured with the detection unit and that is basis for the extraction of the vital sign information. Thus, the illumination unit operates at a certain illumination frequency (usually > 50 Hz so that the human eye does not notice the flickering of the illumination unit). Then, the detection unit is controlled to capture frames when the illumination unit is powered off and no disturbing high energy therapy light is present. Thereby, the detection unit can capture images at ambient light conditions. Thus, the extraction of vital sign information based thereupon is comparable to the extraction of vital sign information from images captured at ambient light, which has been demonstrated before. If, e.g., the flashing frequency of the illumination unit (being part of the configuration of the illumination unit) is known, the sampling frequency (frame rate) of the detection unit may be adapted accordingly.

In a preferred embodiment the illumination unit includes a first light source for emitting phototherapy light and a second light source for emitting light of a frequency spectrum suitable for remote photoplethysmography and the control unit is configured to control the first light source and the second light source to alternatingly illuminate the region of interest. Then, the detection unit can capture images under controlled illumination operating at wavelengths different from the spectrum of the high energy therapy light. Thus, the negative effects of the high energy phototherapy light on the RPPG vital sign extraction can be mitigated. The extraction of vital sign information based on dedicated illumination with a light source that emits a light spectrum optimized for camera-based vital signs extraction (IR light, visible light, etc. depending on which vital signs are to be extracted).

In another embodiment, the illumination unit includes an array of LEDs and the control unit is configured to control the array of LEDs to alternatingly illuminate the region of interest with a first subset of LEDs and a second subset of LEDs, said first subset of LEDs corresponding to LEDs for delivering phototherapy, said second subset of LEDs corresponding to LEDs for emitting light of a frequency spectrum suitable for remote photoplethysmography. LEDs for delivering phototherapy may particularly correspond to high energy blue LEDs. For the camera-based vital sign extraction such blue LEDs can have the disadvantage that only reflected light of one specific spectrum can be detected by the detection unit. This results in an input signal that is less suitable for RPPG extraction of vital sign information, i.e. from which the extraction of vital sign information is difficult or impossible. To overcome this deficiency, the array of LEDs includes LEDs that emit light that is, when reflected from the region of interest, suitable for the extraction of vital sign information such as white light. Also light of a specific wavelength may be advantageous if RPPG is to be used for the extraction of the blood oxygenation. Furthermore, it may also be possible to provide more than two subsets of different LEDs.

According to one embodiment, the configuration of the detection unit includes at least one of a range of selectable frame sampling times and a spectral sensitivity.

According to yet another embodiment, the configuration of the illumination unit includes at least one of a range of possible flashing frequencies, a spectrum of the light source for providing phototherapy, and geometry and control information of an array of LEDs included in the illumination unit.

According to yet another embodiment, the detection unit is configured to filter the detected light, in particular by means of a band-stop optical filter for attenuating the spectrum of phototherapy light being used for delivering the phototherapy treatment to the living being. Such a filter can be realized as a digital filter or as an analog filter. Filtering out the therapy light, which may make it more difficult to obtain an input signal suitable for RPPG vital sign extraction, represents an approach to optimize the input signal generated based on the detected light by the detection unit.

### BRIEF DESCRIPTION OF THE DRAWINGS

These and other aspects of the invention will be apparent from and elucidated with reference to the embodiment(s) described hereinafter. In the following drawings
Fig. 1 shows a schematic illustration of a phototherapy apparatus for treating a living being with light according to an aspect of the present invention;
Fig. 2 shows a schematic illustration of a device for remotely obtaining vital sign information of a living being under phototherapy treatment according to an aspect of the present invention;
Fig. 3 shows a schematic illustration of another embodiment of a device according to the present invention;
Fig. 4 illustrates an example of a light structure generated by means of an array of LEDs in an illumination unit of a phototherapy unit and possible control scheme for a detection unit;
Fig. 5 shows an illustration of an exemplary control of a detection unit according to an aspect of the present invention;
Fig. 6 shows an illustration of another control of a detection unit according to an aspect of the present invention; and
Fig. 7 schematically illustrates a method for remotely obtaining vital sign information carried out by a computer program according to another aspect of the present invention.

### DETAILED DESCRIPTION OF THE INVENTION

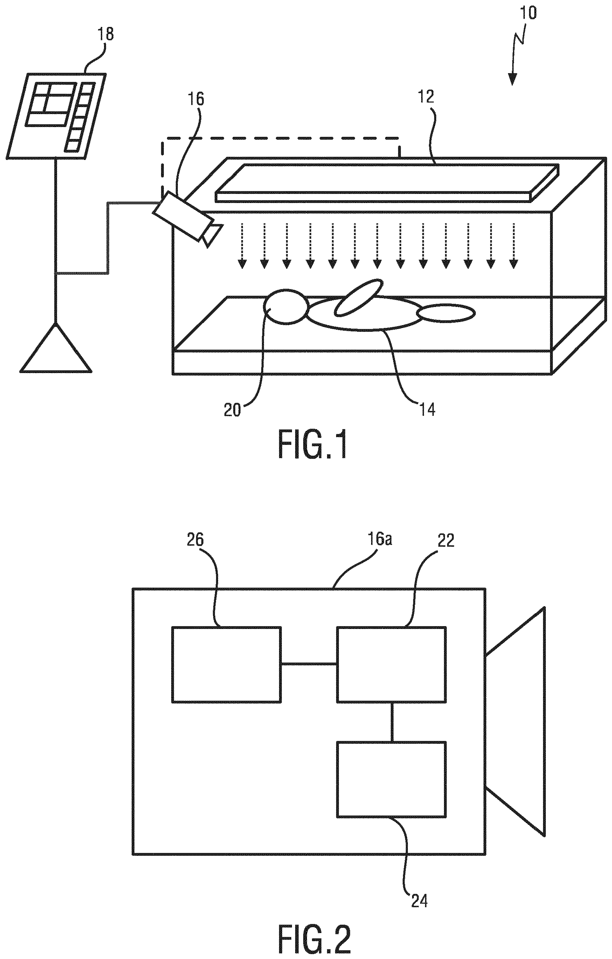

In Fig. 1 a phototherapy apparatus 10 is schematically illustrated. In the field of intensive care for newborns such a phototherapy apparatus 10 is usually integrated with a newborn intensive care unit (NICU) for providing intensive care to babies. When monitoring vital signs of neonates, it is usually desired to not attach contact sensors to their fragile skin but rather rely on camera-based monitoring approaches. Very often premature babies undergo a phototherapy procedure with a narrow band visible light for the treatment of hyperbilirubinemia. While this common therapy lowers the serum bilirubin level, the relatively high intensity of light at a specific wavelength might negatively impact the robustness of camera-based vital signs monitoring. High intensity illumination at a specific (narrow) wavelength (e.g. blue light), which is usually used for phototherapeutic treatment, might negatively impact the robustness of camera-based vital signs monitoring. This is particularly relevant if the parameters, i.e. the configuration, of the detection unit (filters, sampling rate, etc.), i.e. the photosensor of the camera, are not aligned with the phototherapy illumination unit (i.e. the detection unit is not controlled based on the configuration of the illumination unit). For instance, RGB-based methods of RPPG rely on "white spectrum" of ambient illumination. Therefore, strong blue components of the phototherapy light and the absence of other wavelengths could clip pixel values in one color channel and may result in insufficient light in other channels. Moreover, in case the illumination unit of a phototherapy unit is embedded in a matrass, the light entering directly into the lenses of a camera (instead of reflecting from a region of interest of the living being) could cause an enormous glare effect and might even "blind" sensors in at least one color channel. The negative effect of the light from phototherapy on a camera-based monitoring can be even increased in case those light sources show temporal flickering, or if intensity and spectrum of the phototherapy light is adjusting its intensity (irradiance) depending on the body surface area exposed, duration, etc. The present invention allows overcoming these deficiencies by providing a device for remotely obtaining vital sign information of a living being under phototherapy treatment.

In Fig. 1 a phototherapy apparatus 10 is schematically illustrated. In order to deliver the phototherapy, there is foreseen an illumination unit 12 corresponding to a light source for providing the therapeutic light (phototherapy light) to the subject 14 under treatment. This illumination unit 12 can particularly be represented by an array of LEDs. However, the illumination unit can also be represented by a conventional light bulb, an infrared light source, a fluorescent lamp or another light source in other applications. It is also possible that the illumination unit includes multiple light sources, e.g. a first light source for emitting phototherapy light and a second light source for emitting light of another spectrum (e.g. a spectrum suitable for RPPG). The present invention is not limited to be used in the field of newborn intensive care but may also be applied to other application fields of phototherapy such as the treatment of skin disorders (e.g. psoriasis), sleep disorder and some psychiatric disorders.

There is further illustrated in Fig. 1 a device 16 for remotely obtaining vital sign information of the subject 14 according to an aspect of the present invention. In a preferred embodiment the device 16 corresponds to a camera. This device 16 is usually connected to a user interface 18 for outputting the obtained vital sign information. Usually, the device 16 is also connected to an illumination unit 12 as indicated by the dashed line in Fig. 1. This is, however, not necessarily the case in all embodiments of the present invention.

It is to be understood that other application areas of the present invention may require other arrangements of the illumination unit 12 versus the subject 14, such as an illumination unit integrated in the mattress or arranged at another position. It may, however, also be advantageous to make use of two or more vital sign cameras to obtain a more robust or redundant signal without leaving the general concept of the present invention. This may allow capturing images of a larger area.

The device 16 according to the present invention is oriented versus the living being 14 to allow capturing images of a region of interest 20 of the living being 14. This region of interest 20 may particularly refer to a certain area on the forehead of a human being or to an area on the chest of a human being. Usually, the heart rate and/or the blood oxygenation are extracted by means of RPPG based on images of an area on the forehead or another visible skin area. The respiratory rate is usually extracted by means of motion analysis based on images of the chest or belly of the person. It is to be understood that the illustrated region of interest (20) is not to be understood as limiting the invention. Furthermore, the present invention is not limited to be used with human beings but can also be applied to other living beings such as animals.

Fig. 2 schematically illustrates a first embodiment 16a of a device for remotely obtaining vital sign information of a living being 14 under phototherapy treatment according to an aspect of the present invention in greater detail. This device 16a comprises a detection unit 22 for detecting light of at least one wavelength interval reflected from at least a region of interest 20 of the living being 14 and for generating an input signal from the detected light. The detection unit may particularly correspond to the actual photodetector, i.e. a CMOS or CCD chip of a camera.

The device 16a further includes a processing unit 24 for processing the input signal and deriving vital sign information of said living being 14 from said input signal by use of remote photoplethysmography and/or motion analysis. This processing unit 24 may be represented by a standard microprocessor, e.g. a microcontroller unit, or also by an application specific integrated circuit (ASIC). In the processing unit 24 the input signal obtained by means of the detection unit 22 is evaluated. Thereby, particularly fluctuations in the obtained light intensity of one or more pixel are evaluated over time. This so-called photoplethysmographic waveform (corresponding to the input signal) is used to obtain vital sign information, in particular information on the heart rate, the respiratory rate or the blood oxygen saturation of the living being 14.

The device 16a according to the present invention further includes a control unit 26 for controlling at least one of the detection unit 22 and an illumination unit for providing the phototherapy treatment to the living being 14 based on the configuration of the detection unit 22 and based on the configuration of the illumination unit. This control unit 26 may also be represented by a standard microprocessor. In other embodiments of the present invention two or more of the detection unit 22, the processing unit 24 and the control unit 26 may be included in one or more combined processor or integrated circuit. The control unit 26 considers both the configuration of the detection unit 22 and the configuration of an illumination unit in order to control based thereupon at least one of the detection unit 22 and the illumination unit 12. It may be possible that the configuration of the illumination unit 12 is provided to the control unit 26 prior to the use of the device 16a according to the present invention. Then, this configuration can be hard-coded (i.e. obtained and saved prior to the operation of the device) and no further information is required, i.e. no direct communication between the control unit 26 and the illumination unit 12 is required. In this embodiment the detection unit 22 is controlled based on the (previously known) configuration of the illumination unit 12. It may also be possible that the configuration of the detection unit 22 is known prior to the use of the device. Preferably, however, the control unit 26 is in communication with the detection unit 22 as indicated in Fig. 2, obtains the configuration of the detection unit 22 and controls the detection unit 22. In an embodiment of the invention, in which also the illumination unit 12 is to be controlled, usually a direct communication between the control unit 26 and the illumination unit 12 will be required as further detailed below.

It is a key concept of the present invention that the control unit 26 is configured to control at least one of the detection unit 22 and the illumination unit 12 of a phototherapy apparatus based on the configuration of the detection unit 22 and the illumination unit 12. Thereby, it becomes possible that the negative effects of the therapeutic light on the vital sign extraction are mitigated.

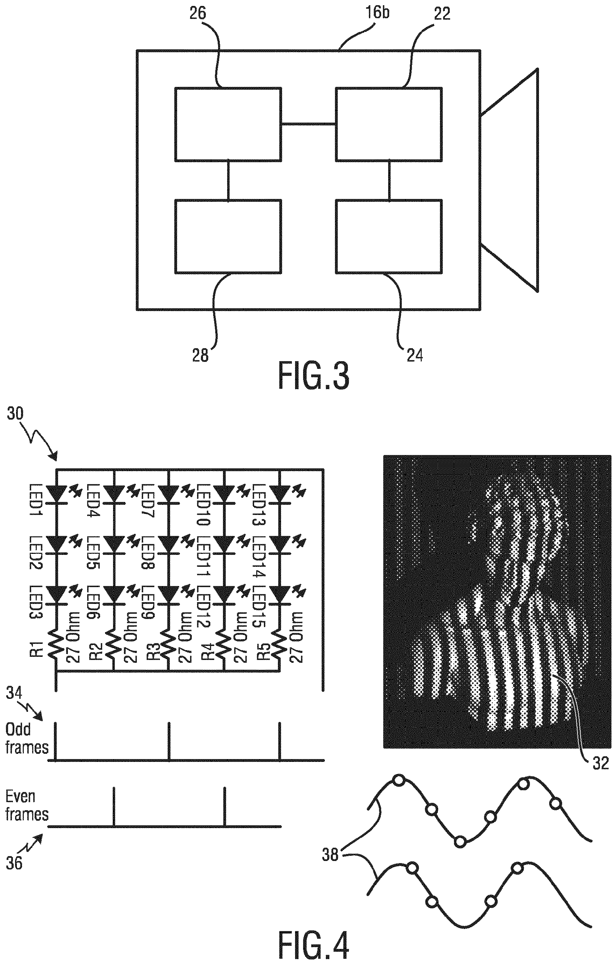

In a preferred embodiment of the device 16b according to the present invention, there is further comprised an illumination unit interface 28 in addition to the detection unit 22, the processing unit 24 and the control unit 26 as illustrated in Fig. 3. Thereby, it becomes possible to directly control the illumination unit 12 and/or to directly obtain information from the illumination unit 12 (i.e. information on the configuration of the illumination unit 12). Further preferably, the device 16b is in communication with an illumination unit 12 via the illumination unit interface 28, wherein the illumination unit 12 includes an array of LEDs. Certain aspects of the present invention may, however, be equivalently applied to other illumination units that are used for providing phototherapy to a living being 14.

One possible approach for the control of the illumination unit is illustrated in Fig. 4. Therein, the illumination unit 12 includes an array of LEDs 30. This array of LEDs 30 can be controlled to produce structured light, i.e. a light pattern, on the region of interest 20 of the living being 14. One example for such a structured light (or light structure) is illustrated on the right side of Fig. 4. As illustrated, the structured light corresponds to a pattern of lines 32 including light and dark zones. Such a pattern 32 may be obtained by illuminating only some LEDs, in particular some lines of LEDs of the array 30. By producing such a light structure 32 on the region of interest 20, it becomes possible to observe chest or belly expansions caused by respiratory efforts by evaluating observed changes over time of the structure 32 (motion analysis). Thus, the detection unit 22 detects light and captures high contrast images that exhibit a light/dark transition, i.e. a line. If the region of interest is then subject to a movement, e.g. a movement caused by an expansion due to a gasp of the living being, the light structure will also be subject to a movement (in particular the line will be bent). Based on this movement of the light structure as observed by the detection unit 22 it becomes possible to derive the respiratory rate of the living being. Thus, it is possible to analyze a motion or movement in the image and deduce therefrom vital sign information of the living being. It is to be understood that other light structures apart from the illustrated line pattern 32 may also be used in a similar way. Controlling the illumination unit of a phototherapy unit to produce a light structure makes it possible to provide phototherapy and to accurately extract the respiratory rate of the living being simultaneously.

Illuminating only a subset of the LEDs in the array 30 may, however, have the disadvantage that a lower light dose is provided than if all LEDs were used. Further, some parts of the skin may be excluded from the treatment or an uneven dose might be provided. In order to avoid this, the illumination unit 12, i.e. the array of LEDs 30, can be controlled to alternatingly produce a first and a second light structure. For instance, it may be possible to illuminate a first subset 34 of LEDs or lines of LEDs of the array 30 alternatingly with a second subset 36 of LEDs or lines of LEDs of the array 30. The detection unit 22 can then, e.g., be controlled to capture the first light structure (produced by the first subset of LEDs 34, i.e. when the first subset of LEDs 34 is illuminated) in every odd frame and to capture the second light structure in every even frame when the second subset of LEDs 36 is illuminated. The illumination unit 12 is thus controlled to operate at a certain flashing frequency and the detection unit 22 is synchronized with the illumination unit 12. If the detection unit 22 is controlled in this manner, the processing unit 24 may extract the respiratory rate 38 from the movements of the two alternating light patterns with a higher robustness. Thus, the robustness when extracting the respiratory rate 38 from the movements of the light structure on the region of interest could be increased.

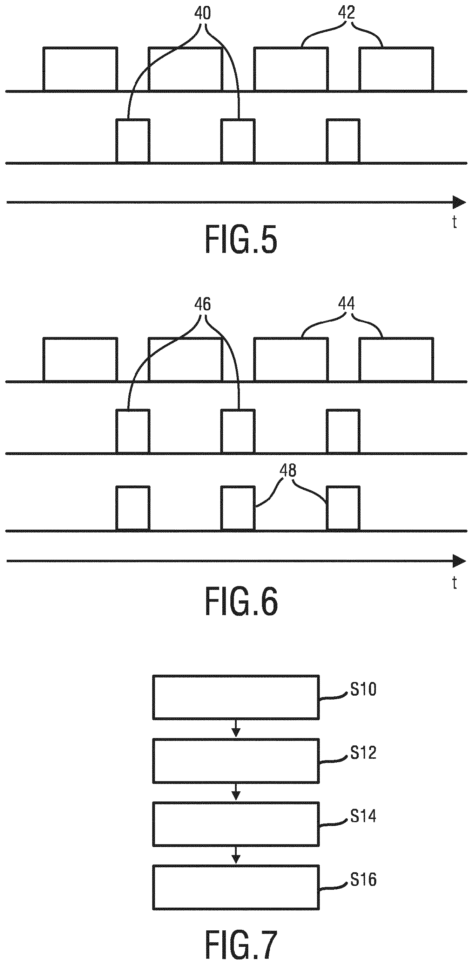

An alternative approach to control the detection unit 22 according to the present invention is illustrated in Fig. 5. Thereby, it is also exploited that the frame sampling time 40, at which the detection unit 22 operates, can be synchronized with a flashing frequency 42 of the illumination unit 12. The illumination unit 12 periodically illuminates the region of interest of the living being. The detection unit 22 is controlled to capture frames (detect light), whenever the illumination unit 12 does not illuminate the region of interest. Thus, the detection unit 22 is controlled to detect reflected ambient light. Therefore, the detection unit 22 is not disturbed by the phototherapy light, i.e. the phototherapy light has no negative impact on the vital sign extraction. It is possible to control the illumination unit 12 by periodically switching it on and off according to a flashing frequency 42. The frame sampling time of the detection unit 40 is adapted accordingly, i.e. the detection unit is controlled to capture frames whenever no phototherapy is provided. Thus, the detection unit 22, i.e. the photosensor, is not blinded by the high intensity therapeutic light of the illumination unit 12. In other embodiments, it may also be possible that only every second frame is captured or that other illumination and detection patterns or frequencies are used.

In order to further optimize the remote monitoring of vital signs of a living being under phototherapy treatment, it may particularly be advantageous to control an illumination unit including an array of LEDs as described above. If this array of LEDs comprises not only one type of LEDs (i.e. LEDs for providing phototherapy) but different types of LEDs, another control approach as illustrated in Fig. 6 can be used. If, e.g. the array of LEDs includes a first subset of LEDs for delivering the phototherapy (e.g. high intensity blue LEDs) and a second subset of LEDs for providing a wide spectrum optimized for remote photoplethysmography (e.g. white LEDs), it is possible to alternatingly illuminate the two subsets. For instance, the high intensity LEDs 44 are illuminated alternatingly with the wide spectrum LEDs 46. The detection unit 22 is controlled to capture frames 48 whenever the second subset of LEDs 46 is switched on and whenever the first subset of LEDs 44 is switched off. In particular, the phototherapy light (blue light) may be emitted most of the time while the white light is only emitted during short periods of time. These short periods of time are used to capture images of the region of interest 20 of the living being 14. The detection frequency of the detection unit 22 (i.e. the frame sampling time) is aligned with the illumination frequency of the LEDs of the array of LEDs. Thereby, a living being 14 can receive an adequate amount of phototherapy treatment albeit vital sign information can be extracted from detected light of another spectrum (which is more suitable for the extraction of vital signs than the spectrum of the therapeutic light). It is to be understood that also other arrays of LEDs including multiple different types of LEDs can be controlled equivalently. For instance, for SpO₂ measurements, the illumination unit 12 can combine blue LEDs, white LEDs and/or infrared LEDs in an array of LEDs. Then, the infrared LEDs and/or the white LEDs can be synchronized with the frame sampling time of the detection unit to allow obtaining an input signal for deriving the SpO₂ rate by evaluating the absorption of light of different wavelengths in the region of interest.

Further, an equivalent approach is also possible if the illumination unit includes a first light source for emitting phototherapy light and a second light source for emitting light of another spectrum, in particular a spectrum suitable for RPPG. Then the control unit can also control the two light sources to alternatingly illuminate the region of interest with the two light sources in the same way as outlined above (i.e. at a certain flashing frequency) and the frame sampling time of the detection unit can be adapted accordingly so that only frames are captured when the second light source is powered on. Still further, it is also possible that the illumination unit includes multiple light sources for emitting light of different wavelengths, e.g. for extracting the blood oxygen saturation).

Other embodiments of the invention can combine the above-outlined approaches or parts thereof. For instance, it may be possible to combine a sequentially flashing LED that emits phototherapy light of an illumination unit 12 that can produce a light structure on the region of interest of the living being with a sequentially flashing white LED. Thereby may become possible to acquire the respiratory rate of the living being from evaluating deformations of the observed structured light and also obtain the heart rate along with the blood oxygen saturation of the living being.

It may also be possible that a plurality of LEDs is combined in the illumination unit 12 and the negative effect of the high energy LEDs with respect to the extraction of vital sign information from the captured images is reduced by applying a blocking optical filter, which blocks a certain spectrum corresponding to the spectrum of the provided phototherapy light and allows the rest of the spectrum. Then, the phototherapy light can continuously be applied while the white and/or infrared LEDs may be synchronized with the frame sampling time of the detection unit as described above.

Fig. 7 illustrates a method carried out by a computer program according to the present invention. The method includes the steps of detecting (step S10) light of at least one wavelength interval reflected from at least a region of interest of the living being and generating (step S12) an input signal from the detected light. The method further comprises processing (step S14) the input signal and deriving vital sign information of said living being from said input signal by use of remote photoplethysmography and/or motion analysis. Still further, the method comprises the step of controlling (step S16) at least one of the detection unit and an illumination unit for providing the phototherapy treatment to the living being based on the configuration of the detection and based on the configuration of the illumination unit.

While the invention has been illustrated and described in detail in the drawings and foregoing description, such illustration and description are to be considered illustrative or exemplary and not restrictive; the invention is not limited to the disclosed embodiments. Other variations to the disclosed embodiments can be understood and effected by those skilled in the art in practicing the claimed invention, from a study of the drawings, the disclosure, and the appended claims.

In the claims, the word "comprising" does not exclude other elements or steps, and the indefinite article "a" or "an" does not exclude a plurality. A single element or other unit may fulfill the functions of several items recited in the claims. The mere fact that certain measures are recited in mutually different dependent claims does not indicate that a combination of these measures cannot be used to advantage.

A computer program may be stored/distributed on a suitable non-transitory medium, such as an optical storage medium or a solid-state medium supplied together with or as part of other hardware, but may also be distributed in other forms, such as via the Internet or other wired or wireless telecommunication systems.

Any reference signs in the claims should not be construed as limiting the scope.

## Claims

1. Device (16) for remotely obtaining vital sign information of a living being (14) under phototherapy treatment, comprising:
a detection unit (22) for detecting light of at least one wavelength interval reflected from at least a region of interest (20) of the living being (14) and for generating an input signal from the detected light;
a processing unit (24) for processing the input signal and deriving vital sign information of said living being (14) from said input signal by use of remote photoplethysmography and/or motion analysis;
an illumination unit interface for communicating with an illumination unit (12) for providing the phototherapy treatment to the living being (14); and
a control unit (26) for controlling at least one of the detection unit (22) and the illumination unit (12) based on the configuration of the detection unit (22) and based on the configuration of the illumination unit (12).

2. Device (16) as claimed in claim 1, wherein
the illumination unit (12) includes an array of LEDs (30); and
the control unit (26) is configured to control the array of LEDs (30) to produce a light structure (32) on the region of interest (20) of the living being (14).

3. Device (16) as claimed in claim 2, wherein the processing unit (24) is configured to
determine changes of the light structure (32) on the region of interest (20) of the living being (14) based on the detected light; and
derive the respiratory rate of the living being (14) based on evaluating the determined changes of the light structure (32).

4. Device (16) as claimed in claim 1, wherein
the illumination unit (12) includes an array of LEDs (30); and
the control unit (26) is configured to control the array of LEDs (30) to produce a recurrent first light structure alternating with a recurrent second light structure on the region of interest (20) of the living being (14), said alternating light structures in particular alternating at an alternation frequency >50 Hz.

5. Device (16) as claimed in claim 4, wherein
the control unit (26) is further configured to control the detection unit (22) to operate at a frame sampling time corresponding to the alternation frequency of the alternating light structures.

6. Device (16) as claimed in claim 1, wherein
the illumination unit (12) includes an array of LEDs (30); and
the control unit (26) is configured to control the array of LEDs (30) to periodically illuminate the region of interest (20) of the living being (14) with at least a subset of LEDs in said array of LEDs (30).

7. Device (16) as claimed in claim 1, wherein
the control unit (26) is configured to control the detection unit (22) to operate at a frame sampling time (40) being adapted to a flashing frequency (42) of the illumination unit (12).

8. Device (16) as claimed in claim 7, wherein
the illumination unit (12) includes a first light source for emitting phototherapy light and a second light source for emitting light of a frequency spectrum suitable for remote photoplethysmography; and
the control unit (26) is configured to control the first light source and the second light source to alternatingly illuminate the region of interest.

9. Device (16) as claimed in claim 7, wherein
the illumination unit (12) includes an array of LEDs (30); and
the control unit (26) is configured to control the array of LEDs (30) to alternatingly illuminate the region of interest (20) with a first subset of LEDs (44) and a second subset of LEDs (46), said first subset of LEDs (44) corresponding to LEDs for delivering phototherapy, said second subset of LEDs (46) corresponding to LEDs for emitting light of a frequency spectrum suitable for remote photoplethysmography.

10. Device (16) as claimed in claim 1, wherein the configuration of the detection unit (22) includes at least one of
a range of selectable frame sampling times; and
a spectral sensitivity.

11. Device (16) as claimed in claim 1, wherein the configuration of the illumination unit (12) includes at least one of
a range of possible flashing frequencies;
a spectrum of the light source for providing phototherapy; and
geometry and control information of an array of LEDs (30) included in the illumination unit (12).

12. Device (16) as claimed in claim 1, wherein
the detection unit (22) is configured to filter the detected light, in particular by means of a band-stop optical filter for attenuating the spectrum of phototherapy light being used for delivering the phototherapy treatment to the living being (14).

13. Phototherapy apparatus (10) for treating a living being (14) with light, comprising:
an illumination unit (12) for providing phototherapy treatment to a living being (14);
a device (16) for remotely obtaining vital sign information of the living being (14) as claimed in claim 1; and
a user interface (18) for outputting the obtained vital sign information.

14. Computer program comprising program code means for causing a computer to carry out the steps of a method for remotely obtaining vital sign information of a living being (14) under phototherapy treatment when said computer program is carried out on the computer, said method comprising the steps of:
detecting (S10) with a detection unit (22) light of at least one wavelength interval reflected from at least a region of interest (20) of the living being (14) and generating (S12) an input signal from the detected light;
processing (S14) the input signal and deriving vital sign information of said living being (14) from said input signal by use of remote photoplethysmography and/or motion analysis;
communicating via an illumination unit interface with an illumination unit (12) for providing the phototherapy treatment to the living being (14); and
controlling (S16) at least one of the detection unit (22) and the illumination unit (12) for providing the phototherapy treatment to the living being (14) based on the configuration of the detection unit (22) and based on the configuration of the illumination unit (12).

## Patentansprüche

1. Vorrichtung (16), um Vitalzeicheninformationen eines Lebewesens (14) unter Phototherapiebehandlung rechnerfern zu erhalten, umfassend:
eine Detektionseinheit (22), um Licht von mindestens einem Wellenlängenintervall zu detektieren, das von zumindest einem interessierenden Bereich (20) des Lebewesens (14) reflektiert wird, und um ein Eingangssignal aus dem detektierten Licht zu erzeugen;
eine Verarbeitungseinheit (24), um das Eingangssignal zu verarbeiten und Vitalzeicheninformationen des Lebewesens (14) aus dem Eingangssignal unter Anwendung von Fern-Photoplethysmographie und/oder Bewegungsanalyse abzuleiten;
eine Beleuchtungseinheit-Schnittstelle zur Kommunikation mit einer Beleuchtungseinheit (12), um die Phototherapiebehandlung für das Lebewesen (14) bereitzustellen; sowie
eine Steuereinheit (26), um zumindest die Detektionseinheit (22) oder die Beleuchtungseinheit (12) auf der Konfiguration der Detektionseinheit (22) basierend und auf der Konfiguration der Beleuchtungseinheit (12) basierend zu steuern.

2. Vorrichtung (16) nach Anspruch 1, wobei
die Beleuchtungseinheit (12) ein Array von LEDs (30) enthält; und
die Steuereinheit (26) so konfiguriert ist, dass sie das Array von LEDs (30) so steuert, dass eine Lichtstruktur (32) auf dem interessierenden Bereich (20) des Lebewesens (14) erzeugt wird.

3. Vorrichtung (16) nach Anspruch 2, wobei die Verarbeitungseinheit (24) so konfiguriert ist, dass sie
Veränderungen der Lichtstruktur (32) auf dem interessierenden Bereich (20) des Lebewesens (14) aufgrund des detektierten Lichts ermittelt; und
die Atemfrequenz des Lebewesens (14) anhand von Auswertungen der ermittelten Veränderungen der Lichtstruktur (32) ableitet.

4. Vorrichtung (16) nach Anspruch 1, wobei
die Beleuchtungseinheit (12) ein Array von LEDs (30) enthält; und
die Steuereinheit (26) so konfiguriert ist, dass sie das Array von LEDs (30) so steuert, dass eine sich wiederholende erste Lichtstruktur im Wechsel mit einer sich wiederholenden zweiten Lichtstruktur auf dem interessierenden Bereich (20) des Lebewesens (14) erzeugt wird, wobei die abwechselnden Lichtstrukturen insbesondere bei einer Wechselfrequenz von >50 Hz alternieren.

5. Vorrichtung (16) nach Anspruch 4, wobei
die Steuereinheit (26) des Weiteren so konfiguriert ist, dass sie die Detektionseinheit (22) so steuert, dass diese bei einer Frame-Sampling-Zeit entsprechend der Wechselfrequenz der abwechselnden Lichtstrukturen arbeitet.

6. Vorrichtung (16) nach Anspruch 1, wobei
die Beleuchtungseinheit (12) ein Array von LEDs (30) enthält, und
die Steuereinheit (26) so konfiguriert ist, dass sie das Array von LEDs (30) so steuert, dass dieses den interessierenden Bereich (20) des Lebewesens (14) mit zumindest einer Teilmenge von LEDs in dem Array von LEDs (30) periodisch beleuchtet.

7. Vorrichtung (16) nach Anspruch 1, wobei
die Steuereinheit (26) so konfiguriert ist, dass sie die Detektionseinheit (22) so steuert, dass diese bei einer Frame-Sampling-Zeit (40) arbeitet, die an eine Blinkfrequenz (42) der Beleuchtungseinheit (12) angepasst ist.

8. Vorrichtung (16) nach Anspruch 7, wobei
die Beleuchtungseinheit (12) eine erste Lichtquelle zur Abstrahlung von Phototherapielicht und eine zweite Lichtquelle zur Abstrahlung von Licht eines zur Fern-Photoplethysmographie geeigneten Frequenzspektrums enthält; und
die Steuereinheit (26) so konfiguriert ist, dass sie die erste Lichtquelle und die zweite Lichtquelle so steuert, dass diese den interessierenden Bereich abwechselnd beleuchten.

9. Vorrichtung (16) nach Anspruch 7, wobei
die Beleuchtungseinheit (12) ein Array von LEDs (30) enthält; und
die Steuereinheit (26) so konfiguriert ist, dass sie das Array von LEDs (30) so steuert, dass der interessierende Bereich (20) mit einer ersten Teilmenge von LEDs (44) und einer zweiten Teilmenge von LEDs (46) abwechselnd beleuchtet wird, wobei die erste Teilmenge von LEDs (44) LEDs zur Bereitstellung von Phototherapie entspricht und die zweite Teilmenge von LEDs (46) LEDs zur Abstrahlung von Licht eines zur Fern-Photoplethysmographie geeigneten Frequenzspektrums entspricht.

10. Vorrichtung (16) nach Anspruch 1, wobei die Konfiguration der Detektionseinheit (22) mindestens einen/eine der folgenden enthält:
einen Bereich auswählbarer Frame-Sampling-Zeiten; sowie
eine Spektralempfindlichkeit.

11. Vorrichtung (16) nach Anspruch 1, wobei die Konfiguration der Beleuchtungseinheit (12) mindestens einen/eine der folgenden enthält:
einen Bereich möglicher Blinkfrequenzen;
ein Spektrum der Lichtquelle zur Phototherapiebereitstellung; sowie
Geometrie- und Steuerungsinformationen eines Arrays von LEDs (30), die in der Beleuchtungseinheit (12) enthalten sind.

12. Vorrichtung (16) nach Anspruch 1, wobei
die Detektionseinheit (22) so konfiguriert ist, dass sie das detektierte Licht, insbesondere mit Hilfe eines optischen Bandsperrfilters, filtert, um das Spektrum von Phototherapielicht abzuschwächen, das zur Bereitstellung der Phototherapiebehandlung für das Lebewesen (14) eingesetzt wird.

13. Phototherapievorrichtung (10) zur Behandlung eines Lebewesens (14) mit Licht, umfassend:
eine Beleuchtungseinheit (12), um eine Phototherapiebehandlung für ein Lebewesen (14) bereitzustellen;
eine Vorrichtung (16), um Vitalzeicheninformationen des Lebewesens (14) rechnerfern zu erhalten nach Anspruch 1; sowie
eine Benutzerschnittstelle (18) zur Ausgabe der erhaltenen Vitalzeicheninformationen.

14. Computerprogramm mit Programmcodemitteln, um einen Computer zu veranlassen, dass er die Schritte eines Verfahrens zum rechnerfernen Empfang von Vitalzeicheninformationen eines Lebewesens (14) unter Phototherapiebehandlung ausführt, wenn das Computerprogramm auf dem Computer ausgeführt wird, wobei das Verfahren die folgenden Schritte umfasst, wonach:
mit Hilfe einer Detektionseinheit (22) Licht von mindestens einem Wellenlängenintervall detektiert wird (S10), das von zumindest einem interessierenden Bereich (20) des Lebewesens (14) reflektiert wird, und ein Eingangssignal aus dem detektierten Licht erzeugt wird (S12);
das Eingangssignal verarbeitet wird (S14) und Vitalzeicheninformationen des Lebewesens (14) aus dem Eingangssignal unter Anwendung von Fern-Photoplethysmographie und/oder Bewegungsanalyse abgeleitet werden;
über eine Beleuchtungseinheit-Schnittstelle mit einer Beleuchtungseinheit (12) kommuniziert wird, um die Phototherapiebehandlung für das Lebewesen (14) bereitzustellen; und
zumindest die Detektionseinheit (22) oder die Beleuchtungseinheit (12) so gesteuert wird (S16), dass für das Lebewesen (14) die Phototherapiebehandlung auf der Konfiguration der Detektionseinheit (22) basierend und auf der Konfiguration der Beleuchtungseinheit (12) basierend bereitgestellt wird.

## Revendications

1. Dispositif (16) pour l'obtention à distance d'informations sur les signes vitaux d'un être vivant (14) sous traitement par photothérapie, comprenant :
une unité de détection (22) pour détecter la lumière d'au moins un intervalle de longueur d'onde réfléchie à partir d'au moins une région d'intérêt (20) de l'être vivant (14) et pour générer un signal d'entrée à partir de la lumière détectée ;
une unité de traitement (24) pour traiter le signal d'entrée et en déduire des informations sur les signes vitaux dudit être vivant (14) à partir dudit signal d'entrée en utilisant la photopléthysmographie à distance et/ou une analyse de mouvement ;
une interface d'unité d'éclairage pour communiquer avec une unité d'éclairage (12) pour assurer le traitement par photothérapie de l'être vivant (14) ; et
une unité de commande (26) pour commander au moins l'un parmi l'unité de détection (22) et l'unité d'éclairage (12) sur la base de la configuration de l'unité de détection (22) et sur la base de la configuration de l'unité d'éclairage (12).

2. Dispositif (16) selon la revendication 1, dans lequel
l'unité d'éclairage (12) inclut une série de LED (30) ; et
l'unité de commande (26) est configurée pour commander la série de LED (30) pour produire une structure lumineuse (32) sur la région d'intérêt (20) de l'être vivant (14).

3. Dispositif (16) selon la revendication 2, dans lequel l'unité de traitement (24) est configurée pour
déterminer des changements de la structure lumineuse (32) sur la région d'intérêt (20) de l'être vivant (14) sur la base de la lumière détectée ; et
déduire la fréquence respiratoire de l'être vivant (14) sur la base de l'évaluation des changements déterminés de la structure lumineuse (32).

4. Dispositif (16) selon la revendication 1, dans lequel
l'unité d'éclairage (12) inclut une série de LED (30) ; et
l'unité de commande (26) est configurée pour commander la série de LED (30) pour produire une première structure lumineuse récurrente alternant avec une deuxième structure lumineuse récurrente sur la région d'intérêt (20) de l'être vivant (14), lesdites structures lumineuses alternées alternant en particulier à une fréquence d'alternance > 50 Hz.

5. Dispositif (16) selon la revendication 4, dans lequel
l'unité de commande (26) est en outre configurée pour commander l'unité de détection (22) pour fonctionner dans un temps d'échantillonnage cadre correspondant à la fréquence d'alternance des structures lumineuses alternées.

6. Dispositif (16) selon la revendication 1, dans lequel
l'unité d'éclairage (12) inclut une série de LED (30) ; et
l'unité de commande (26) est configurée pour commander la série de LED (30) pour éclairer périodiquement la région d'intérêt (20) de l'être vivant (14) avec au moins un sous-groupe de LED dans ladite série de LED (30).

7. Dispositif (16) selon la revendication 1, dans lequel
l'unité de commande (26) est configurée pour commander l'unité de détection (22) pour fonctionner dans un temps d'échantillonnage cadre (40) qui est adapté à une fréquence de clignotement (42) de l'unité d'éclairage (12).

8. Dispositif (16) selon la revendication 7, dans lequel
l'unité d'éclairage (12) inclut une première source de lumière pour émettre une lumière de photothérapie et une deuxième source de lumière pour émettre une lumière à un spectre de fréquence approprié pour une photopléthysmographie à distance ; et
l'unité de commande (26) est configurée pour commander la première source de lumière et la deuxième source de lumière pour éclairer en alternance la région d'intérêt.

9. Dispositif (16) selon la revendication 7, dans lequel
l'unité d'éclairage (12) inclut une série de LED (30) ; et
l'unité de commande (26) est configurée pour commander la série de LED (30) pour éclairer en alternance la région d'intérêt (20) avec un premier sous-groupe de LED (44) et un deuxième sous-groupe de LED (46), ledit premier sous-groupe de LED (44) correspondant aux LED servant à délivrer la photothérapie, ledit deuxième sous-groupe de LED (46) correspondant aux LED pour émettre une lumière d'un spectre de fréquence approprié pour une photopléthysmographie à distance.

10. Dispositif (16) selon la revendication 1, dans lequel la configuration de l'unité de détection (22) inclut au moins l'un parmi
une série de temps d'échantillonnage cadres ; et
une sensibilité spectrale.

11. Dispositif (16) selon la revendication 1, dans lequel la configuration de l'unité d'éclairage (12) inclut au moins l'un parmi
une série de fréquences de clignotement possibles ;
un spectre de la source de lumière pour assurer une photothérapie ; et
des informations sur la géométrie et la commande d'une série de LED (30) incluses dans l'unité d'éclairage (12).

12. Dispositif (16) selon la revendication 1, dans lequel
l'unité de détection (22) est configurée pour filtrer la lumière détectée, en particulier au moyen d'un filtre optique coupe-bande pour atténuer le spectre de lumière de photothérapie qui est utilisée pour délivrer le traitement par photothérapie à l'être vivant (14).

13. Appareil de photothérapie (10) pour traiter un être vivant (14) par la lumière, comprenant :
une unité d'éclairage (12) pour assurer un traitement par photothérapie d'un être vivant (14) ;
un dispositif (16) pour obtenir à distance des informations sur les signes vitaux de l'être vivant (14) selon la revendication 1 ; et
une interface utilisateur (18) pour émettre les informations sur les signes vitaux obtenues.

14. Programme informatique comprenant des moyens de codage de programme pour entraîner l'exécution par un ordinateur des étapes d'un procédé pour obtenir à distance des informations sur les signes vitaux d'un être vivant (14) sous traitement par photothérapie lorsque ledit programme informatique est exécuté sur l'ordinateur, ledit procédé comprenant les étapes de :
détection (S10) avec une unité de détection (22) de la lumière d'au moins un intervalle de longueur d'onde réfléchie à partir d'au moins une région d'intérêt (20) de l'être vivant (14) et pour générer (S12) un signal d'entrée à partir de la lumière détectée ;
traitement (S14) du signal d'entrée et déduction des informations sur les signes vitaux dudit être vivant (14) à partir dudit signal d'entrée en utilisant la photopléthysmographie à distance et/ou une analyse de mouvement ;
communication via une interface d'unité d'éclairage avec une unité d'éclairage (12) pour assurer le traitement par photothérapie de l'être vivant (14) ; et
commande (S16) d'au moins l'un parmi l'unité de détection (22) et l'unité d'éclairage (12) pour assurer le traitement par photothérapie de l'être vivant (14) sur la base de la configuration de l'unité de détection (22) et sur la base de la configuration de l'unité d'éclairage (12).
